(19) Europäisches Patentamt / European Patent Office / Office européen des brevets

(11) **EP 2 769 668 B1**

(12) **FASCICULE DE BREVET EUROPEEN**

(45) Date de publication et mention
de la délivrance du brevet:
**13.10.2021 Bulletin 2021/41**

(51) Int Cl.:
*A61B 5/00* (2006.01)          *A61B 5/024* (2006.01)
*A61N 1/39* (2006.01)          *A61N 1/37* (2006.01)

(21) Numéro de dépôt: **14156180.3**

(22) Date de dépôt: **21.02.2014**

(54) **Ensemble pour le diagnostic adaptatif de l'insuffisance cardiaque moyennant des classifieurs et un arbre de décision booléen**

System zur adaptiven Diagnose von Herzinsuffizienz mittels Klassifikatoren und eines booleschen Entscheidungsbaums

System for the adaptive diagnosis of chronic heart failure using classifying means and a boolean decision tree

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.02.2013 FR 1351664**

(43) Date de publication de la demande:
**27.08.2014 Bulletin 2014/35**

(73) Titulaire: **Sorin CRM SAS**
**92140 Clamart Cedex (FR)**

(72) Inventeurs:
• **Dumont, Jérôme**
**92320 Chatillon (FR)**

• **Baumann, Oliver**
**Southampton, SO19 4BZ (GB)**

(74) Mandataire: **Grünecker Patent- und Rechtsanwälte**
**PartG mbB**
**Leopoldstraße 4**
**80802 München (DE)**

(56) Documents cités:
**US-A1- 2002 029 002          US-A1- 2006 010 090**
**US-A1- 2006 184 496          US-A1- 2012 157 856**

**Description**

L'invention concerne les "dispositifs médicaux actifs" tels que définis par la directive 93/42/CE du 14 juin 1993 du Conseil des communautés européennes, plus particulièrement les dispositifs implantables de stimulation cardiaque, resynchronisation et/ou défibrillation destinés au diagnostic et au traitement des troubles du rythme cardiaque.

Cette définition inclut également les dispositifs actifs, implantés ou non, à visée purement diagnostique - par exemple les systèmes externes de suivi à domicile des patients (*home monitoring* ou *remote monitoring*), mettant en oeuvre une liaison sans fil avec un appareil d'interrogation à distance disposé près du porteur du dispositif, qui est activé à intervalles périodiques, par exemple quotidiennement, pour télécharger les données recueillies par ce dispositif et les transmettre pour analyse à un site distant de télésurveillance.

L'invention concerne plus particulièrement le diagnostic d'événements précoces de décompensation cardiaque (événements ci-après désignés "événements délétères" ou AE, *Adverse Events*) au moyen d'algorithmes d'analyse de signaux recueillis par le dispositif.

La détection précoce de la décompensation cardiaque est un problème complexe, reflété chez un patient par de multiples paramètres physiologiques, qui sont susceptibles d'être évalués et analysés pour délivrer éventuellement une alerte. En particulier :

- la présence de fluide dans les poumons, susceptible de produire une diminution de l'amplitude respiratoire ainsi que de l'impédance transtho-racique ;
- une accélération de la fréquence respiratoire, notamment de manière plus précoce à l'effort ;
- une prise de poids et une fatigue ressentie par le patient, conduisant celui-ci à faire moins d'exercice, ou des exercices moins intenses, avec donc une fréquence cardiaque maximale moindre ;
- une diminution de la fraction d'éjection lors des efforts ;
- une diminution de la variabilité du rythme cardiaque ;
- des évolutions de divers paramètres morphologiques des signaux d'électrogramme endocavitaire (EGM) et/ou d'accélération endocardiaque (EA) ;
- la présence d'épisodes de fibrillation atriale (FA), surtout de FA conduite, qui est également une source potentielle importante de décompensation cardiaque.

On constate que l'on peut disposer ainsi d'un très grand nombre de données liées à l'état clinique du patient, dérivées de l'activité électrique du myocarde, notamment de signaux EGM recueillis par des électrodes implantées, de signaux EA ou de bioimpédance cardiaque, ou encore de signaux reflétant les variations de divers paramètres tels que fréquence cardiaque, fréquence et amplitude ventilatoires, activité, etc. obtenus à partir de mesures effectuées par des capteurs d'activité (capteur G accélérométrique) et/ou des capteurs physiologiques (capteur MV de ventilation-minute).

L'évolution de ces données peut être déterminée par exemple chaque jour. Toutefois, pris séparément, l'analyse des indicateurs quotidiens ne donne pas forcément de très bons résultats, car ils sont généralement peu spécifiques.

En revanche, une combinaison judicieuse de ces indicateurs peut pallier, au moins partiellement, ce manque de spécificité. De très nombreux index peuvent être élaborés à cet effet. Ainsi, le EP 1 867 360 A2 (ELA Medical) propose de recouper diverses informations issues de capteurs MV et G avec un signal d'accélération endocardiaque ou de bioimpédance cardiaque. Des algorithmes d'analyse produisent des index de risque de décompensation cardiaque, et des moyens d'analyse croisée délivrent un indicateur composite d'alerte préventive, sur différents niveaux, en fonction des index produits par les algorithmes.

Concrètement, les algorithmes de prévention de décompensation cardiaque utilisés jusqu'à présent génèrent toutefois un nombre assez élevé de fausses alertes.

Il s'agit généralement de faux positifs, qui sont sans intérêt pour le médecin mais qui risquent d'inquiéter inutilement le patient. Les conséquences de fausses alertes répétées peuvent toutefois être importantes lorsque le dispositif non seulement délivre une alerte (fonction de diagnostic), mais en outre modifie son fonctionnement pour s'adapter à l'amélioration ou aggravation supposée de l'état du patient, par exemple par reprogrammation de certaines des fonctions du dispositif ou modification automatique de seuils de déclenchement.

Le paramétrage des différents critères de déclenchement de l'alerte est généralement choisi de manière à privilégier la sensibilité de déclenchement de l'alerte, par ajustement de divers paramètres fixes (seuils), incrémentaux (pourcentages minima ou maxima d'augmentation), ou encore de métarègles analysant l'évolution des index et de leur combinaison sur plusieurs jours.

Le US 2006/0010090 A1 décrit ainsi un système expert comprenant une pluralité de seuils correspondant aux diverses informations recueillies par un dispositif implantable, avec possibilité pour le praticien de modifier ces seuils en fonctions d'indications fournies par le système expert à partir notamment de l'historique du patient. Il est ainsi possible d'accroitre la sélectivité du système et éviter des déclenchements d'alarme intempestifs. Cette proposition ne prend toutefois pas en considération le fait que la sensibilité et la spécificité (c'est-à-dire la sélectivité de l'analyse) sont généralement considérées comme deux notions antagonistes, dans la mesure où avec les algorithmes connus une augmentation de sensibilité s'accompagne généralement d'une moindre spécificité - avec corrélativement une augmentation des risques de fausses alertes ("faux positifs"). Inversement, si l'on rend les critères d'alerte plus stricts, on réduit les cas de faux positifs, mais avec le risque de ne pas déclencher d'alerte dans des cas critiques ("faux négatifs"), situation qu'il convient d'éviter autant que faire se peut.

Le but de l'invention est de mettre à disposition une technique permettant d'adapter un algorithme de prévention de la décompensation cardiaque, de manière évolutive pour un patient donné en fonction des fausses alertes et/ou des alertes manquées précédemment rencontrées chez ce patient, afin de ne plus les reproduire.

Plus précisément, et comme on le verra plus en détail, l'invention propose une technique d'adaptation d'un tel algorithme qui tient compte à la fois :

- des faux positifs (les erreurs les plus fréquentes), par une mise à jour directe du dispositif après que ces faux positifs ont été identifiés comme tels par un médecin, cette mise à jour se faisant par un ajustement simple de paramètres utilisés par l'algorithme ; et
- des faux négatifs (erreurs moins fréquentes mais très problématiques), par une adaptation plus profonde de l'algorithme, à partir d'un panel de patients de référence dont le profil est conservé dans une base de données, et qui auront été identifiés comme ayant eu des comportements similaires aux patients courants.

On connait, par exemple d'après le US 2005/0216067 A1 (Pacesetter, Inc.) des algorithmes mettant en oeuvre des combinaisons linéaires de divers paramètres.

Le US 2003/0055461 A1 (CPI) prévoit par ailleurs de calculer au moins certains des facteurs de pondération d'une telle combinaison linéaire en utilisant des données historiques reflétant une corrélation entre le paramètre physiologique pondéré et l'état clinique plus ou moins aggravé du patient.

Les algorithmes linéaires sont cependant sous-optimaux lorsqu'il s'agit d'évaluer un risque de survenue d'une décompensation cardiaque, dans la mesure où il n'y a *a priori* aucune raison pour que le système que l'on souhaite représenter suive une relation linéaire. Par exemple, la présence d'épisodes de fibrillation auriculaire (FA) est un indicateur important, qui doit faire l'objet d'une pondération forte lorsque la durée quotidienne de ces épisodes est importante ; mais en revanche, l'absence de FA ne signifie pas que le risque de décompensation soit faible. Ou encore, si un patient produit rarement des efforts, il est inutile de pondérer fortement les variables recherchant des augmentations du rythme à l'effort, puisque ces variables ne seront pas fiables.

Pour pallier ces limitations, l'invention propose de mettre en oeuvre une méthode non-linéaire (à la différence de celles décrites dans les documents précités), à base d'arbre de décision, c'est-à-dire une méthode basée sur un algorithme dans lequel les différentes données recueillies sont comparées à des seuils respectifs hiérarchisés entre eux. Un tel algorithme peut être formalisé par une équation booléenne, comprenant une succession de termes - correspondant aux comparaisons des différents paramètres par rapport aux seuils (dépassement/non-dépassement du seuil respectif) - qui sont combinés par une suite de ET logiques, et dont le résultat est un résultat binaire de type "alerte/pas d'alerte".

Un tel algorithme non-linéaire basé sur un arbre de décision présente le double avantage :

- d'être mieux adapté que les algorithmes linéaires à la détection précoce des risques de décompensation cardiaque, car il répond mieux aux divers cas de décompensation qui ont pu être cliniquement observés ; et
- facilement adaptable dans le temps, en partant d'un algorithme générique qui est ensuite affiné au cours du temps en fonction de données spécifiques observées pour le patient concerné.

Plus précisément, l'invention propose un ensemble comprenant, de manière en elle-même connue d'après le US 2006/0010090 A1 précité :

a) un dispositif médical actif de type stimulateur, resynchroniseur, défibrillateur et/ou appareil à visée diagnostique, comportant :

- des moyens d'acquisition, de traitement et de mémorisation de données d'état clinique d'un patient courant porteur du dispositif médical, ces données étant des données multimodales liées à l'activité cardiaque et évaluées sur des intervalles de temps prédéterminés successifs ;
- des moyens classifieurs, aptes à analyser lesdites données pour les comparer à un ensemble de seuils et générer en réponse un indicateur d'alerte ou d'absence d'alerte ;
- des moyens de mémorisation des indicateurs successifs générés par les moyens classifieurs ;
- des moyens de mise à jour desdits seuils en réponse à une commande externe, et

b) des premiers moyens externes au dispositif médical, comprenant :

- des moyens aptes à transmettre au dispositif médical ladite commande externe pour ladite mise à jour des seuils, et

c) des seconds moyens externes au dispositif médical, comprenant :

- une base de données de patients de référence, mémorisant pour chaque patient de référence un ensemble desdites données d'état clinique avec des marqueurs associés indiquant la présence ou l'absence d'un évènement délétère ; et
- des moyens pour recevoir en entrée un ensemble des données d'état clinique du patient courant, avec des marqueurs associés indiquant la présence ou l'absence d'un évènement délétère.

De façon caractéristique de l'invention :

- lesdits seuils sont des seuils successifs hiérarchisés selon une configuration booléenne d'arbre de décision ;
- les premiers moyens externes comprennent des moyens de supervision aptes, pour chacun des indicateurs générés par les moyens classifieurs :

    i) à recevoir en entrée un marqueur indiquant la présence ou l'absence d'un évènement délétère observé au cours de l'intervalle de temps correspondant à l'indicateur respectif,
    ii) à recevoir en entrée une notation (vrai/faux positif/négatif) indiquant, en fonction de la valeur correspondante du marqueur, si l'alerte ou l'absence d'alerte de l'indicateur représente : un vrai positif, un vrai négatif, un faux positif, ou bien un faux négatif, et
    iii) à associer à chaque indicateur le marqueur et la notation qui lui correspond,

- les premiers moyens externes comprennent en outre, pour la correction des faux positifs :

    - des moyens aptes, en présence d'une notation de faux positif, à transmettre aux moyens classifieurs du dispositif médical une commande de décalage des valeurs des seuils ayant déclenché l'alerte, et

- les seconds moyens externes comprennent en outre, pour la correction des faux négatifs :

    - des moyens sélecteurs, pour extraire de la base de données un groupe réduit de patients de référence significatifs, par des comparaisons entre les données d'état clinique du patient courant et les données d'état clinique des patients de référence, ces comparaisons étant effectuées distinctement pour les données dont le marqueur associé indique la présence d'un évènement délétère et pour celles dont le marqueur associé indique l'absence d'un évènement délétère ;
    - des moyens de création d'arbre de décision, aptes à définir lesdits seuils successifs de l'arbre de décision et hiérarchiser ces seuils selon ladite configuration booléenne ; et
    - des moyens aptes à transmettre aux moyens classifieurs du dispositif médical l'arbre de décision ainsi créé.

Les données d'état clinique peuvent notamment comprendre des données évaluées sur un intervalle de temps prédéterminé à partir de variables parmi : moyenne de la fréquence cardiaque au repos et à l'exercice ; moyenne de la fréquence cardiaque maximale atteinte à chaque exercice ; moyenne de la fréquence respiratoire au repos et à l'exercice ; moyenne de la fréquence respiratoire maximale atteinte à chaque exercice ; temps passé en fibrillation auriculaire ; amplitude ventilatoire à l'exercice et au repos ; quantité d'exercice mesurée par un accéléromètre ; variabilité du rythme cardiaque ; variabilité de paramètres morphologiques de signaux d'électrogramme endocavitaire ; et/ou variabilité de paramètres morphologiques d'accélération endocardiaque.

On va maintenant décrire un exemple de mise en oeuvre de l'invention, en référence aux dessins annexés où les mêmes références désignent d'une figure à l'autre des éléments identiques ou fonctionnellement semblables.

La Figure 1 est un exemple d'arbre de décision permettant d'analyser les diverses données d'état clinique du patient pour générer en réponse un indicateur d'alerte ou d'absence d'alerte.

La Figure 2 est un diagramme schématique, sous forme de blocs fonctionnels, des divers éléments constitutifs de l'algorithme de l'invention, avec les divers moyens permettant de le faire évoluer au cours du temps de manière à mieux l'adapter au patient courant.

Les Figures 3a et 3b illustrent la technique de mise à jour directe par une modification des seuils de l'arbre de décision permettant d'augmenter la spécificité de l'alerte.

La Figure 4 illustre la manière de sélectionner un panel de patients de référence les plus proches du patient courant, distinctement pour les périodes avec évènements délétères et celles sans évènement délétère.

On va maintenant décrire un exemple de mise en oeuvre de la technique de l'invention.

De façon générale, l'invention est mise en oeuvre par des moyens principalement logiciels, au moyen d'algorithmes appropriés exécutés par exemple par des microcontrôleurs ou processeurs numériques de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées au sein d'un même logiciel.

En ce qui concerne le dispositif médical employé dans le cadre de cette technique, l'invention peut être mise en oeuvre avec une programmation appropriée du logiciel de commande d'un implant cardiaque connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés. L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Bien que l'on parlera par la suite d'"'implant", l'invention n'est pas limitée à ce type de dispositif et peut être mise en oeuvre avec des dispositifs externes, par exemple des dispositifs à visée essentiellement diagnostique recueillant et mémorisant diverses informations destinées à être analysées pour évaluer l'état clinique d'un patient.

Comme on l'a indiqué en introduction, le but de l'invention est de mettre en oeuvre une technique d'analyse de données d'état clinique d'un patient porteur d'un dispositif médical, généralement un dispositif implanté, permettant de conduire à la délivrance (ou non) d'une alerte préventive de décompensation cardiaque.

*Arbre de décision*

Plus précisément, la technique de l'invention repose sur une analyse de type non-linéaire de ces données d'état clinique, plus précisément une analyse des données par comparaison à un ensemble de seuils successifs hiérarchisés selon une configuration booléenne d'arbre de décision, conduisant à la délivrance d'un indicateur binaire "alerte/pas d'alerte" comme résultat.

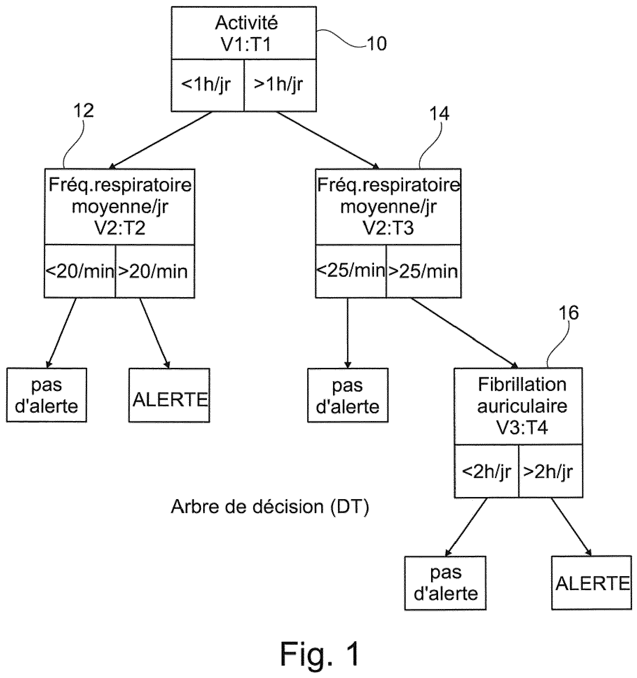

La Figure 1 illustre un exemple d'un tel arbre de décision (DT, *Décision Tree*), qui montre la manière de hiérarchiser les comparaisons aux différents seuils, chaque comparaison déterminant de façon binaire le franchissement ou non du seuil considéré et, selon le résultat, conduisant à un branchement conditionnel vers d'autres comparaisons à d'autres seuils, et ainsi de suite jusqu'à la production en réponse de l'indicateur final d'alerte ou d'absence d'alerte.

Dans l'exemple simplifié illustré, la première donnée testée (bloc 10) est le niveau d'activité quotidien V1, qui est comparé à un premier seuil T1, inférieur ou supérieur à 1 heure d'activité par jour. Pour une activité V1 inférieure à ce seuil T1, le test suivant (bloc 12) portera sur la fréquence respiratoire moyenne quotidienne V2, testée par rapport à un seuil T2, inférieur ou supérieur à 20 respirations par minute. Si ce seuil n'est pas franchi, l'algorithme produit un indicateur "pas d'alerte", en revanche si le seuil est dépassé l'indicateur sera "alerte" (c'est-à-dire une alerte préventive de décompensation cardiaque, signalant la présence - selon l'algorithme - d'un risque de survenue à court terme d'un épisode de décompensation cardiaque).

Dans le cas d'une activité V1 supérieure au seuil T1, la fréquence respiratoire V2 est comparée (bloc 14) à un seuil T3, différent du seuil T2 précédent. Ce seuil est par exemple de 25 respirations par minute et s'il n'est pas franchi l'indicateur produit est "pas d'alerte". Dans le cas contraire, un test supplémentaire est opéré (bloc 16) sur une troisième donnée V3, à savoir la durée quotidienne moyenne passée en fibrillation auriculaire, qui est comparée à un seuil T4. Si ce seuil n'est pas franchi, l'indicateur sera "pas d'alerte", dans le cas contraire une "alerte" sera produite.

Un tel arbre de décision peut être formalisé par une relation booléenne qui, dans l'exemple décrit ci-dessus, sera :

$$\text{Alerte} = (V1 < T1 \ \& \ V2 > T2) + (V1 > T1 \ \& \ V2 > T3 \ \& \ V3 > T4)$$

Un tel arbre de décision peut être généralisé à l'analyse d'un très grand nombre de données d'état clinique du patient, en elles-mêmes relativement complexes et riches en informations, et dont on donnera plus bas divers exemples. Toutes ces données sont liées à l'activité cardiaque et hémodynamique du patient et évaluées sur des intervalles de temps prédéterminés successifs, par exemple quotidiennement.

Concrètement, un algorithme tel que celui que l'on vient de décrire produit un indicateur "alerte" ou "pas d'alerte" à partir des données d'état clinique analysé automatiquement, mais cet indicateur ne reflète pas nécessairement la réalité physiologique et clinique, c'est-à-dire la présence ou l'absence d'un véritable évènement délétère (AE, *Adverse Event*), confirmé du point de vue clinique.

Selon le cas, pour chaque indication d'alerte ou d'absence d'alerte produite par l'arbre de décision, on peut se trouver en présence d'un vrai/faux positif/négatif, avec les quatre possibilités résumées par le tableau 1 suivant :

*Tableau 1*

|  | **Alerte** | **Pas d'alerte** |
|---|---|---|
| **AE** | vrai positif | faux négatif |
| **Pas d'AE** | faux positif | vrai négatif |

L'objet de l'invention est, dans un tel contexte :

- d'adapter les différents seuils d'un tel arbre de décision de manière dynamique, en fonction des faux positifs avérés, de manière à améliorer la sélectivité de l'algorithme au fil de ces adaptations successives ; et
- de construire ou reconstruire cet arbre, notamment en présence de faux négatifs avérés, c'est-à-dire définir de manière automatisée et rationnelle l'enchainement des branches de l'arbre tel que décrit par l'équation booléenne correspondante, ainsi que le niveau des différents seuils.

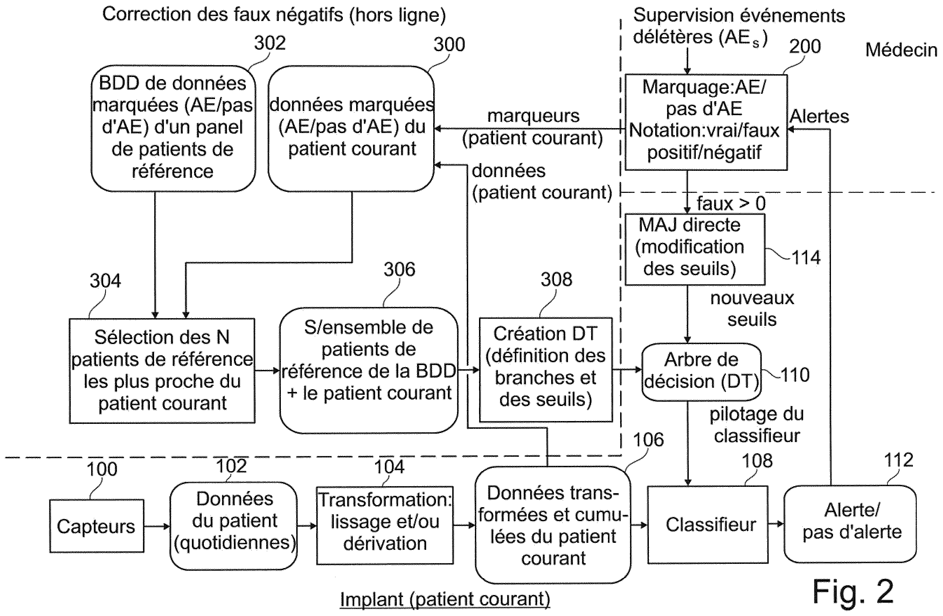

La Figure 2 explicite sous forme de schéma par blocs la technique proposée par l'invention, permettant d'adapter l'arbre de décision (configuration des branches et niveau des seuils) de manière à réduire faux positifs et faux négatifs, et améliorer ainsi les performances de l'algorithme d'alerte préventive de décompensation cardiaque.

Sur la Figure 2, on a séparé les fonctions exécutées distinctement :

- par l'implant, c'est-à-dire le dispositif porté par le patient courant recueillant et traitant les différentes données d'état clinique de façon continue : blocs 100 à 114 ;
- par un module de supervision par un médecin : bloc 200 ;
- par un système de correction des faux négatifs mettant en oeuvre une base de données et un moteur d'apprentissage : blocs 300 à 308.

*Opérations exécutées au sein de l'implant*

L'implant recueille un certain nombre de données d'état du patient au moyen de capteurs (bloc 100), qui peuvent notamment délivrer :

- des signaux d'activité électrique du myocarde, notamment un électrogramme endocavitaire (EGM) ;
- des signaux d'activité hémodynamique, tels qu'un signal d'accélération endocardiaque (EA) ou de bioimpédance cardiaque ;
- des signaux reflétant les variations de divers paramètres (fréquence cardiaque, fréquence et amplitude ventilatoire, activité, etc.) lors d'une alternance de phase d'effort et de récupération, déterminées par des capteurs physiques (accéléromètre G) ou physiologiques (ventilation-minute MV) donnant une indication de ce niveau d'activité ;
- d'autres signaux encore tels que capteurs de pression, de saturation d'oxygène dans le sang, etc., fonction de l'état hémodynamique du patient.

Ces données sont recueillies sur un intervalle de temps prédéterminé, par exemple journalier, et traitées (bloc 102) pour produire des données d'état clinique telles que par exemple :

- moyenne de la fréquence cardiaque au repos et à l'exercice ;
- moyenne de la fréquence cardiaque maximale atteinte à chaque exercice ;
- moyenne de la fréquence respiratoire au repos et à l'exercice ;
- moyenne de la fréquence respiratoire maximale atteinte à chaque exercice ;
- temps passé en fibrillation auriculaire ;

- amplitude ventilatoire à l'exercice et au repos ;
- quantité d'exercice mesurée par un accéléromètre ;
- variabilité du rythme cardiaque ;
- variabilité de paramètres morphologiques des signaux EGM ; et/ou
- variabilité de paramètres morphologiques des signaux EA.

Ces données d'état clinique font l'objet d'un traitement (bloc 104) par exemple de débruitage par lissage temporel sur des fenêtres de taille variable, et éventuellement de transformation par dérivation de manière à déterminer leur variation temporelle, par exemple en calculant des pentes par régression linéaire ou en comparant une moyenne à court terme (7 jours) à une moyenne à long terme (30 jours). Une même variable peut être séparément d'une part lissée et d'autre part dérivée, résultant en deux données transformées différentes issues de la même variable mais représentées de deux façons différentes.

On constitue ainsi pour un patient donné (ci-après "patient courant") une base de données (bloc 106) de séries temporelles de diverses données d'état clinique transformées (issues de la transformation par le bloc 104), avec une donnée par jour et par variable.

Ces données sont ensuite analysées par un classifieur 108 opérant sous le contrôle d'un arbre de décision 110 tel que celui qu'on a décrit plus haut en exemple avec la Figure 1. Ce classifieur utilise l'arbre de décision 110 enregistré dans l'implant pour répartir les dernières données transformées dans les classes "alerte" ou "pas d'alerte" (bloc 112).

Les alertes successives ainsi générées à intervalles réguliers, typiquement chaque jour, sont ultérieurement analysées (bloc 200) par un médecin qui supervise l'historique des données enregistrées dans l'implant pour y détecter la présence ou non d'événements délétères AE.

Il s'agit à ce stade d'opérer une classification supervisée, c'est-à-dire une classification où la survenue effective des AE est connue et documentée, et où l'on sait donc où se trouvent ces AE dans les séries temporelles produites et enregistrées (bloc 106) par l'implant.

Lorsqu'un AE est avéré par le médecin, celui-ci examine si une alerte correspondante a été ou non générée par le classifieur 108 et attribue une notation "vrai positif" si l'implant a effectivement produit une alerte, et "faux négatif" dans le cas contraire. Il examine également la série d'alertes qui ont été produites par le classifieur : si effectivement la période considérée révélait un risque élevé de décompensation cardiaque pour le patient (AE survenue ou imminente), alors le médecin attribue une notation "vrai positif" ; dans le cas contraire, une notation "faux positif".

À chaque AE avérée et à chaque alerte générée et enregistrée par l'implant est ainsi associée une notation vrai/faux positif/négatif, selon le tableau 1 ci-dessus.

*Mise à jour des seuils de l'arbre de décision*

En cas de faux positif avéré à ce stade à partir de l'analyse effectuée par le médecin, il est possible et avantageux de procéder à une mise à jour directe de l'arbre de décision, par modification des seuils de l'arbre de décision (bloc 114) de manière à éviter la production ultérieure de faux positifs de même type.

Les seuils mis à jour sont les seuils ayant déclenché une alerte, qui sont recherchés et modifiés de façon automatique (bloc 114) par l'algorithme de mise à jour directe.

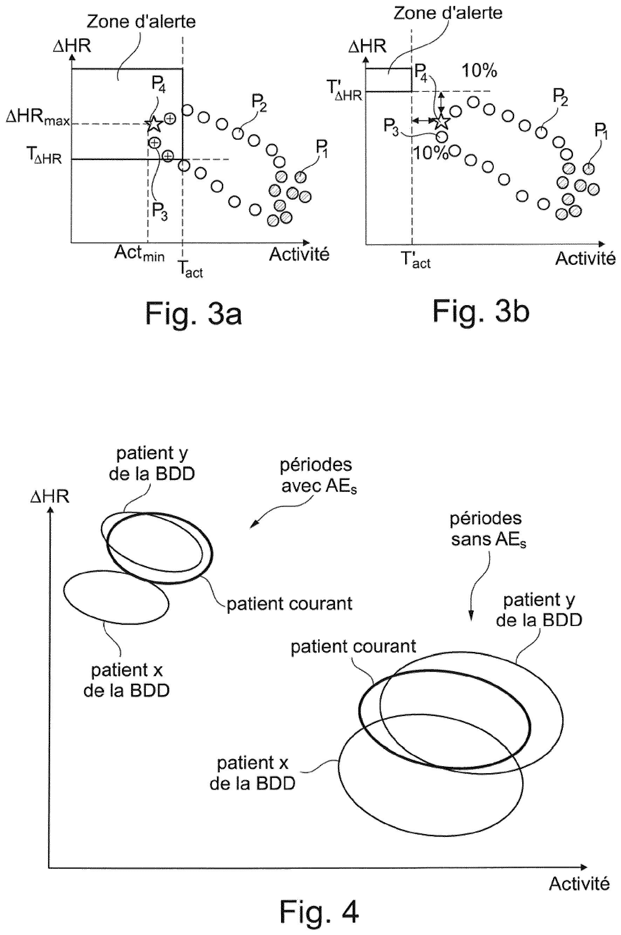

Un exemple de mise à jour de seuils est illustré par les Figures 3a et 3b, qui correspondent à la représentation de deux données concernées, à savoir la durée quotidienne passée en activité ("Activité") et la variabilité du rythme cardiaque ("HR").

Dans le cas de la Figure 3a, qui correspond à la situation ayant déclenché une fausse alerte (faux positif), les données quotidiennes sont représentées par un nuage de points, les points $P_1$ hachurés correspondant à une situation initiale du patient avec activité élevée et faible variabilité, reflétant un bon état clinique. Ensuite (points $P_2$ non hachurés), l'activité diminue et la variabilité du rythme augmente, les points entrant dans la zone d'alerte définie par les seuils $T_{act}$ et $T_{\Delta HR}$ : points $P_3$ marqués d'un "+" et point $P_4$, le plus éloigné des seuils courants $T_{act}$ et $T_{\Delta HR}$ (point de coordonnées $\Delta HR_{max}$, $Act_{min}$. La recherche du point $P_4$ peut être effectuée notamment par normalisation des données puis calcul de distance euclidienne.

Si le médecin a déterminé que l'alerte produite aux points $P_3$ et $P_4$ est une fausse alerte (faux positif), alors en réponse à cette détermination les seuils $T_{act}$ et $T_{\Delta HR}$ sont modifiés, par exemple décalés de manière à laisser subsister une marge de 10 % par rapport au point $P_4$.

Les nouveaux seuils deviennent, comme illustré sur la Figure 3b : pour l'activité : $T_{act}$ = 90 % de Actmin et pour $\Delta HR$ : $T'_{\Delta HR}$ = 110 % $\Delta HR_{max}$.

La zone d'alerte se trouve ainsi redéfinie à une distance suffisante du point $P_4$ pour ne pas produire ultérieurement de faux positifs.

Ce type de correction par simple décalage des seuils n'est en revanche pas applicable en cas de faux négatif, car l'algorithme ne peut pas connaitre le terme de l'équation booléenne définissant l'arbre de décision qui aurait dû déclencher l'alerte et qui ne l'a pas fait. Il n'est pas non plus souhaitable de modifier globalement l'ensemble des seuils, car l'impact sur la spécificité pourrait être important.

Dans ce cas, il est préférable de recréer un arbre de décision.

*Création/recréation de l'arbre de décision*

Cette recréation de l'arbre de décision est opérée hors ligne, en comparant (bloc 300) les données du patient courant marquées (AE/pas d'AE) et notées (vrai/faux positif/négatif) par le médecin, à une base de données marquées (AE/pas d'AE) d'un panel significatif de patients de référence, dont les données d'état clinique ont été précédemment enregistrées et analysées et dont on connait les dates des décompensations cardiaques, ce qui a permis de marquer (AE/pas d'AE) chacune de ces données d'état clinique.

Ces données respectives (du patient courant et du panel de patients de référence) sont appliquées à un module de sélection (bloc 304) des N patients de référence les plus proches du patient courant, c'est-à-dire de sélection de patients ayant par rapport au patient courant : i) des profils de données d'état clinique de base similaires et ii) un comportement comparable face à la survenue d'un AE. La sélection de ces N patients de référence peut être opérée par exemple en analysant une métrique de type distance de Bregman, cette sélection étant opérée de façon distincte pour les périodes avec AE et les périodes sans AE.

La Figure 4 illustre un tel exemple de sélection, dans un domaine qui correspond à la représentation de la durée quotidienne passée en activité ("Activité") et de la variabilité du rythme cardiaque ("ΔHR").

Pour chaque patient de référence de la base de données, un rang de classement par rapport au patient courant lui est attribué par un algorithme comprenant les étapes suivantes :

- calcul des distances entre chaque AE du patient courant et chaque AE des patients de référence ;
- pour chaque patient de référence et pour chaque AE du patient courant, sélection de la seule distance la plus faible ;
- détermination pour chaque AE du patient courant du rang des patients de référence ; et
- évaluation du rang moyen entre le patient courant et les patients de référence (moyenne des rangs de chacun des AE).

La sélection des patients de référence est basée sur la comparaison des données "AE" et "pas d'AE" du patient courant, représentées par des gaussiennes multivariées, avec les données des patients de référence. La distance de Bregman est utilisée comme distance entre les distributions, ce qui permet de prendre en compte les covariances en plus des moyennes.

Les patients sont ensuite classés suivant l'inverse de leur distance par rapport au patient courant, distinctement pour les périodes avec AE et pour les périodes sans AE.

Plus précisément, les périodes "AE" correspondent par exemple aux données mesurées J jours avant la survenue de l'AE (par exemple J = 10 jours), les données "pas d'AE" étant les données mesurées sur des périodes de M mois, par exemple M = 2 mois sauf pendant le mois précédent un AE.

Suivant le nombre de périodes avec AE ou sans AE, plusieurs cas particuliers peuvent se présenter :

- si l'on trouve pour un patient de référence plusieurs périodes avec AE ou plusieurs périodes sans AE, on prendra en compte la plus proche du patient courant ;
- dans le cas de plusieurs périodes avec AE chez le patient courant, on effectuera la moyenne des rangs des différents AE, mais on prendra toujours en compte la dernière période de référence.

Une fois qu'un rang a été attribué à chaque patient de référence, pour les périodes avec/sans AE, les rangs "période avec AE" et "période sans AE" sont moyennés, et les patients de référence sont classés, puis un nombre réduit N est sélectionné (bloc 306), par exemple N = 15 à 20 patients de référence, qui seront considérés comme étant les plus proches du patient courant.

Les données correspondant aux patients ainsi sélectionnés vont servir à créer un arbre de décision (bloc 308). La création de l'arbre de décision consiste à définir i) les différentes branches de l'arbre (définition de l'équation booléenne) ainsi que ii) le niveau des différents seuils auxquels sont comparées les données d'état clinique respectives.

L'arbre de décision est créé de façon automatique par un module d'apprentissage comprenant N entrées (les différentes données d'état clinique possibles) et deux sorties (l'indicateur binaire alerte/pas d'alerte), en excluant éventuellement certaines séries de données lorsque l'on n'est pas sûr que les données observées soient liées à la survenue d'un AE (par exemple dans une période relativement lointaine avant la survenue de l'AE).

L'arbre de décision peut être créé par exemple par application d'un algorithme de Breiman tel que celui décrit par : Breiman L, Friedman JH, Olshen R et Stone C, *Classification and Regression Trees,* Boca Raton, FL. Lors de l'apprentissage, des poids sont affectés aux vrais/faux positifs/négatifs de manière à instaurer une fréquence de fausses alertes à ne pas dépasser, par exemple pas plus de deux faux positifs par année et par patient.

L'arbre de décision ainsi créé peut alors être introduit par télémétrie dans l'implant (arbre de décision 110), par exemple à l'occasion d'une mise à jour du logiciel lors d'une visite chez le médecin, et l'on peut escompter que sa mise en oeuvre permettra de réduire autant les faux négatifs que les faux positifs.

**Revendications**

1.  Un ensemble pour le diagnostic de l'insuffisance cardiaque, comprenant :

    a) un dispositif médical actif de type stimulateur, resynchroniseur, défibrillateur et/ou appareil à visée diagnostique, comportant :

    • des moyens (100-106) d'acquisition, de traitement et de mémorisation de données d'état clinique d'un patient courant porteur du dispositif médical, ces données étant des données multimodales liées à l'activité cardiaque et évaluées sur des intervalles de temps prédéterminés successifs ;
    • des moyens classifieurs (108), aptes à analyser lesdites données pour les comparer à un ensemble de seuils et générer en réponse un indicateur (112) d'alerte ou d'absence d'alerte ;
    • des moyens de mémorisation des indicateurs successifs générés par les moyens classifieurs ;
    • des moyens (114) de mise à jour desdits seuils en réponse à une commande externe, et

    b) des premiers moyens externes au dispositif médical, comprenant :

    • des moyens aptes à transmettre au dispositif médical ladite commande externe pour ladite mise à jour des seuils, et

    c) des seconds moyens externes au dispositif médical, comprenant :

    • une base de données (302) de patients de référence, mémorisant pour chaque patient de référence un ensemble desdites données d'état clinique avec des marqueurs associés indiquant la présence ou l'absence d'un évènement délétère ; et
    • des moyens pour recevoir en entrée un ensemble des données d'état clinique du patient courant (300), avec des marqueurs associés indiquant la présence ou l'absence d'un évènement délétère,

    ensemble **caractérisé en ce que** :

    - lesdits seuils sont des seuils successifs hiérarchisés selon une configuration booléenne d'arbre de décision (110) ;
    - les premiers moyens externes comprennent des moyens de supervision (200) aptes, pour chacun des indicateurs générés par les moyens classifieurs :

    i) à recevoir en entrée un marqueur (AE/pas d'AE) indiquant la présence ou l'absence d'un évènement délétère observé au cours de l'intervalle de temps correspondant à l'indicateur respectif,
    ii) à recevoir en entrée une notation (vrai/faux positif/négatif) indiquant, en fonction de la valeur correspondante du marqueur, si l'alerte ou l'absence d'alerte de l'indicateur représente : un vrai positif, un vrai négatif, un faux positif, ou bien un faux négatif, et
    iii) à associer à chaque indicateur le marqueur et la notation qui lui correspond,

    - les premiers moyens externes comprennent en outre, pour la correction des faux positifs :

    • des moyens aptes, en présence d'une notation de faux positif, à transmettre aux moyens classifieurs du dispositif médical une commande de décalage des valeurs des seuils ayant déclenché l'alerte, et

    - les seconds moyens externes comprennent en outre, pour la correction des faux négatifs :

    • des moyens sélecteurs (304), pour extraire de la base de données un groupe réduit de patients de référence significatifs (306), par des comparaisons entre les données d'état clinique du patient courant et les données d'état clinique des patients de référence, ces comparaisons étant effectuées distinctement pour les données dont le marqueur associé indique la présence d'un évènement délétère et pour celles dont le marqueur associé indique l'absence d'un évènement délétère ;
• des moyens (308) de création d'arbre de décision, aptes à définir lesdits seuils successifs de l'arbre de décision et hiérarchiser ces seuils selon ladite configuration booléenne ; et
• des moyens aptes à transmettre aux moyens classifieurs du dispositif médical l'arbre de décision ainsi créé.

**2.** L'ensemble de la revendication 1, dans lequel les données d'état clinique comprennent des données évaluées sur un intervalle de temps prédéterminé à partir de variables parmi : moyenne de la fréquence cardiaque au repos et à l'exercice ; moyenne de la fréquence cardiaque maximale atteinte à chaque exercice ; moyenne de la fréquence respiratoire au repos et à l'exercice ; moyenne de la fréquence respiratoire maximale atteinte à chaque exercice ; temps passé en fibrillation auriculaire ; amplitude ventilatoire à l'exercice et au repos ; quantité d'exercice mesurée par un accéléromètre ; variabilité du rythme cardiaque ; variabilité de paramètres morphologiques de signaux d'électrogramme endocavitaire ; et/ou variabilité de paramètres morphologiques d'accélération endocardiaque.

## Patentansprüche

**1.** System zur adaptiven Diagnose von chronischer Herzinsuffizienz, einschließlich:

a) ein aktives medizinisches Gerät wie einen Stimulator, einen Resynchronisator, einen Defibrillator und/oder ein Diagnosegerät, das Folgendes umfasst:

• Mittel (100-106) zum Erfassen, Verarbeiten und Speichern von klinischen Zustandsdaten eines aktuellen Patienten, der die medizinische Vorrichtung trägt, wobei die Daten multimodale Daten sind, die sich auf die Herzaktivität beziehen und über aufeinanderfolgende vorbestimmte Zeitintervalle ausgewertet werden;
• Klassifizierungsmittel (108), die in der Lage sind, die Daten zu analysieren, um sie mit einem Satz von Schwellenwerten zu vergleichen und als Reaktion darauf einen Alarm- oder Nicht-Alarm-Indikator (112) zu erzeugen;
• Mittel zum Speichern aufeinanderfolgender Indikatoren, die von den Klassifizierungsmitteln erzeugt werden;
• Mittel (114) zum Aktualisieren der Schwellenwerte als Reaktion auf einen externen Befehl, und

b) eine erste Einrichtung außerhalb des Medizinprodukts, die Folgendes umfasst:

• Mittel, die geeignet sind, die externe Anforderung der Schwellenwertaktualisierung an das medizinische Gerät zu übertragen, und

c) eine zweite Einrichtung außerhalb des Medizinprodukts, die Folgendes umfasst:

• eine Datenbank (302) von Referenzpatienten, die für jeden Referenzpatienten einen Satz der klinischen Zustandsdaten mit zugehörigen Markern speichert, die das Vorhandensein oder Fehlen eines schädlichen Ereignisses anzeigen; und
• Mittel zum Empfangen eines Satzes von klinischen Zustandsdaten des aktuellen Patienten (300) als Eingabe, mit zugehörigen Markern, die das Vorhandensein oder Fehlen eines schädlichen Ereignisses anzeigen,
**dadurch gekennzeichnet, dass**:

- die genannten Schwellenwerte sind aufeinanderfolgende hierarchische Schwellenwerte gemäß einer booleschen Entscheidungsbaumkonfiguration (110);
- die ersten externen Mittel Überwachungsmittel (200) umfassen, die in der Lage sind, für jeden der von den Klassifizierungsmitteln erzeugten Indikatoren:

i) als Eingabe einen Marker (AE/No AE) zu empfangen, der das Vorhandensein oder Nichtvorhandensein eines schädlichen Ereignisses anzeigt, das während des dem jeweiligen Indikator entsprechenden Zeitintervalls beobachtet wurde,
ii) als Eingabe eine Notation (wahr/falsch positiv/negativ) darüber zu erhalten, ob die Warnung oder das Ausbleiben einer Warnung des Indikators ein wahres Positiv, ein wahres Negativ, ein falsches Positiv oder ein falsches Negativ darstellt, und

(iii) jeden Indikator mit der entsprechenden Markierung und Notation zu verknüpfen,

- die ersten externen Mittel umfassen darüber hinaus die Korrektur von Fehlalarmen:
- Mittel, die in der Lage sind, bei Vorliegen einer falsch-positiven Bewertung an die Klassifizierungsmittel des medizinischen Geräts eine Aufforderung zur Verschiebung der Werte der Schwellenwerte, die den Alarm ausgelöst haben, zu übermitteln, und

- die zweiten externen Mittel umfassen ferner die Korrektur von falschnegativen Ergebnissen:

• Selektionsmittel (304), um aus der Datenbank eine reduzierte Gruppe signifikanter Referenzpatienten (306) durch Vergleiche zwischen den klinischen Zustandsdaten des aktuellen Patienten und den klinischen Zustandsdaten der Referenzpatienten zu extrahieren, wobei solche Vergleiche getrennt für Daten, deren zugehöriger Marker das Vorhandensein eines schädlichen Ereignisses anzeigt, und für Daten, deren zugehöriger Marker das Fehlen eines schädlichen Ereignisses anzeigt, durchgeführt werden;
• Mittel (308) zur Erzeugung eines Entscheidungsbaums, die in der Lage sind, die aufeinanderfolgenden Schwellenwerte des Entscheidungsbaums zu definieren und diese Schwellenwerte gemäß dem booleschen Muster zu priorisieren; und
• Mittel, die in der Lage sind, den so erstellten Entscheidungsbaum an die Klassifizierungsmittel des Medizinprodukts zu übertragen.

2. System nach Anspruch 1, wobei die Daten zum Gesundheitszustand Daten umfassen, die über ein vorbestimmtes Zeitintervall aus Variablen bewertet wurden, einschließlich: durchschnittliche Herzfrequenz in Ruhe und bei Belastung; durchschnittliche maximale Herzfrequenz, die bei jeder Belastung erreicht wird; durchschnittliche Atemfrequenz in Ruhe und bei Belastung; durchschnittliche Atemfrequenz bei jeder Belastung; durchschnittliche Herzfrequenz in Ruhe und bei Belastung; und durchschnittliche Atemfrequenz bei jeder Belastung: durchschnittliche Herzfrequenz in Ruhe und bei Belastung; durchschnittliche maximale Herzfrequenz, die bei jeder Belastung erreicht wird; durchschnittliche Atemfrequenz in Ruhe und bei Belastung; durchschnittliche maximale Atemfrequenz, die bei jeder Belastung erreicht wird; im Vorhofflimmern verbrachte Zeit; Ventilationsamplitude bei Belastung und in Ruhe; von einem Beschleunigungsmesser gemessenes Ausmaß der Belastung; Variabilität der Herzfrequenz; Variabilität der morphologischen Parameter der endokardialen Elektrogrammsignale; und/oder Variabilität der morphologischen Parameter der endokardialen Beschleunigung.

**Claims**

1. A system for the adaptive diagnosis of chronic heart failure, including:

a) an active medical device such as a stimulator, resynchroniser, defibrillator and/or diagnostic device, comprising :

• means (100-106) for acquiring, processing and storing clinical status data of a current patient carrying the medical device, said data being multimodal data related to cardiac activity and evaluated over successive predetermined time intervals;
• classifier means (108) capable of analysing said data to compare it to a set of thresholds and generating an alert or non-alert indicator (112) in response;
• means for storing successive indicators generated by the classifier means;
• means (114) for updating said thresholds in response to an external command, and

b) first means external to the medical device, comprising :

• means adapted to transmit to the medical device said external request for said threshold update, and

c) second means external to the medical device, comprising :

• a database (302) of reference patients, storing for each reference patient a set of said clinical status data with associated markers indicating the presence or absence of a deleterious event; and
• means for receiving as input a set of clinical condition data of the current patient (300), with associated markers indicating the presence or absence of a deleterious event,

**characterised in that** :

- said thresholds are successive hierarchical thresholds according to a Boolean decision tree configuration (110);
- the first external means comprise supervisory means(200) capable, for each of the indicators generated by the classifier means:

i) to receive as input a marker (AE/No AE) indicating the presence or absence of a deleterious event observed during the time interval corresponding to the respective indicator,
ii) to receive as input a notation (true/false positive/negative) as to whether the alert or lack of alert of the indicator represents: a true - positive, a true negative, a false positive, or a false negative, and
(iii) to associate each indicator with its corresponding marker and notation,

- the first external means further comprise, for the correction of false positives:

• means capable, in the presence of a false positive rating, of transmitting to the classifier means of the medical device a request to shift the values of the thresholds which triggered the alert,
and
the second external means further comprise, for the correction of false negatives:
• selector means (304) for extracting from the database a reduced group of significant reference patients (306) by comparisons between the clinical status data of the current patient and the clinical status data of the reference patients, such comparisons being made separately for data whose associated marker indicates the presence of a deleterious event and for data whose associated marker indicates the absence of a deleterious event;
• decision tree creation means (308) capable of defining said successive thresholds of the decision tree and prioritising these thresholds according to said Boolean pattern; and
• means capable of transmitting the decision tree thus created to the classifier means of the medical device.

2. The system according to claim 1, wherein the health status data comprises data assessed over a predetermined time interval from variables including: average resting and exercising heart rate; average maximum heart rate achieved at each exercise; average resting and exercising respiratory rate; average respiratory rate at each exercise; average resting and exercising heart rate; and average respiratory rate at each exercise: average resting and exercising heart rate; average maximum heart rate achieved at each exercise; average resting and exercising respiratory rate; average maximum respiratory rate achieved at each exercise; time spent in atrial fibrillation; ventilatory amplitude at exercise and at rest; amount of exercise measured by an accelerometer; variability of heart rate; variability of morphological parameters of endocardial electrogram signals; and/or variability of morphological parameters of endocardial acceleration.

Arbre de décision (DT)

# Fig. 1

Correction des faux négatifs (hors ligne)

Supervision événements
délétères (AE$_s$)

Médecin

302

300

200

```
BDD de données
marquées (AE/pas
d'AE) d'un panel
de patients de
référence
```

```
données marquées
(AE/pas d'AE) du
patient courant
```

marqueurs
(patient courant)

```
Marquage:AE/
pas d'AE
Notation:vrai/faux
positif/négatif
```

Alertes

données
(patient courant)

faux > 0

304

306

308

```
MAJ directe
(modification
des seuils)
```

114

```
Sélection des N
patients de référence
les plus proche du
patient courant
```

```
S/ensemble de
patients de
référence de la BDD
+ le patient courant
```

```
Création DT
(définition des
branches et
des seuils)
```

nouveaux
seuils

```
Arbre de
décision (DT)
```

110

pilotage du
classifieur

106

108

112

100

102

104

```
Capteurs
```

```
Données
du patient
(quotidiennes)
```

```
Transformation:
lissage et/ou
dérivation
```

```
Données trans-
formées et cumu-
lées du patient
courant
```

```
Classifieur
```

```
Alerte/
pas d'alerte
```

Implant (patient courant)

Fig. 2

Fig. 3a

Fig. 3b

Fig. 4

**EP 2 769 668 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1867360 A2
- US 20060010090 A1
- US 20050216067 A1
- US 20030055461 A1